# EUROPÄISCHE PATENTSCHRIFT

(11) **EP 1 587 552 B1**
(45) Veröffentlichungstag und Bekanntmachung des Hinweises auf die Patenterteilung: **17.08.2011**
(21) Anmeldenummer: 03767660.8
(22) Anmeldetag: 26.11.2003
(51) Int. Cl.: A61L 2/20

(54) **MIKROTOM-KRYOSTAT UND VERFAHREN ZU SEINER DESINFEKTION**
MICROTOME CRYOSTAT AND METHOD FOR DISINFECTING IT
CRYOSTAT DE MICROTOME ET PROCEDE DE SA DESINFECTION

(30) Priorität: 01.02.2003 DE 10303989
(43) Veröffentlichungstag der Anmeldung: 26.10.2005
(73) Patentinhaber: Microm International GmbH, 69190 Walldorf (DE)
(72) Erfinder: TEPPKE, Dieter, 68723 Schwetzingen (DE)
(74) Vertreter: Weber, Walter
(86) Internationale Anmeldenummer: PCT/EP2003/013267
(87) Internationale Veröffentlichungsnummer: WO 2004/067047

(56) Entgegenhaltungen:
- DE-A- 3 202 627
- US-A- 4 952 370
- US-A- 5 974 811
- US-A1- 2002 139 124

## Beschreibung

Die Erfindung betrifft ein Verfahren zur Desinfektion eines Mikrotom-Kryostaten mit einer Entfrostungsphase, einer Bereitstellung eines dampfförmigen Desinfektionsmittels zur Einwirkung auf die geschlossene Kryostatkammer und einer Einwirkzeit für das Desinfektionsmittel.

Die Erfindung betrifft weiterhin einen Mikrotom-Kryostaten mit einer Kryostatkammer, in der sich ein Mikrotom befindet, und mit einem Kälteerzeuger, umfassend eine Vorrichtung mit einer Einrichtung zur Bereitstellung eines dampfförmigen Desinfektionsmittels und einer Steuerung, die derart ausgebildet ist, daß sie diese Bereitstellung sowie eine Einwirkzeit im Anschluß an eine Entfrostungsphase veranlaßt.

Da mit Mikrotomen oft Gewebeproben geschnitten werden, die mit Keimen infiziert sind, ist eine Desinfektion zum Schutz der Bedienpersonen geboten. Ein Einsprühen ist insbesondere bei Kryostatmikrotomen problematisch, da die Feuchtigkeit wieder aus der Kryostatkammer entfernt werden muß. Verbleibt Feuchtigkeit an einem Kryostatmikrotom, so gefriert diese nach einer Kühlung für den nächsten Arbeitsgang. Dabei können Führungen und sonstiges einfrieren, wodurch das Mikrotom nicht mehr einsatzfähig ist. Oder das Desinfektionsmittel bleibt, beispielsweise bei hochkonzentrierten alkoholischen Lösungen, flüssig in der Kammer und führt zu weiteren Beeinträchtigungen bis hin zur Explosionsgefahr.

Ein Vorschlag zur Lösung dieses Problems durch die US 2002/0139124 A1 besteht darin, eine Desinfektion mit Ozon vorzunehmen. Dies hat jedoch den Nachteil, daß Ozon sehr stark korrosiv wirkt und daher Oberflächen durch Oxidation beschädigt werden. Ein weiterer Nachteil besteht darin, daß Ozonreste schwer entfernbar, giftig und stark brandfördernd sind, teilweise aber auch schon während der Desinfektionsphase aus dem Gerät austreten und dann Bedienpersonen gefährden können.

In dem Firmenprospekt "AS 600 Cryotome" der Firma ANGLIA SCIENTIFIC besteht ein weiterer Vorschlag darin, eine Dekontamination mit UV-Strahlen vorzunehmen. Dies hat jedoch den Nachteil, daß die UV-Strahlen nicht in Schattenbereiche gelangen. Außerdem ist auch die Eindringtiefe der UV-Strahlen in Schnittabfälle oder Probenreste und in mikroskopisch kleine Vertiefungen von Metalloberflächen nicht gegeben, so daß diese Dekontamination unbefriedigend ist.

Ein Vorschlag der eingangs genannten Art ist aus den Firmenprospekten "AS 620 Cryotome" der Firma ANGLIA SCIENTIFIC Instruments LTD sowie aus dem "AS 620 Cryotome Instruction Manual" bekannt. Dabei wird ein Formalinspender erhitzt und die geschlossene Kryostatkammer mit Formalindampf beaufschlagt. Auch hier tritt jedoch das eingangs genannte Problem mit im Kryostaten und insbesondere am Mikrotom verbleibender Feuchtigkeit auf, was dazu führt, daß das Gerät längere Zeit offenstehen muß, um auszutrocknen. Dabei entweicht Desinfektionsmittel, was aus gesundheitlichen Gründen und wegen eventueller Geruchsstörung möglichst vermieden werden sollte. Außerdem ist das Gerät längere Zeit nicht einsatzfähig oder nicht sicher getrocknet.

Der Erfindung liegt daher die Aufgabe zugrunde, ein Verfahren und eine Vorrichtung der eingangs genannten Art derart weiterzubilden, daß eine schnelle und effektive Desinfektion und eine möglichst rasche Wiederinbetriebnahhme eines Mikrotom-Kryostaten bei sicherer Trocknung möglich ist.

Bezüglich des Verfahrens wird die Aufgabe erfindungsgemäß dadurch gelöst, daß nach der Einwirkungszeit in der Kryostatkammer eine Temperaturdifferenz zwischen einem das Mikrotom umfassenden Bereich und einem kälteren Bereich erzeugt und das im kälteren Bereich niedergeschlagene Desinfektionsmittel abgeführt wird.

Bezüglich der Vorrichtung wird die Aufgabe erfindungsgemäß dadurch gelöst, daß die Steuerung derart ausgebildet ist, daß sie nach der Einwirkzeit durch Heizung und/oder Kühlung in der Kryostatkammer die Entstehung einer Temperaturdifferenz zwischen einem das Mikrotom umfassenden Bereich und einem kälteren Bereich veranlaßt und im kälteren Bereich eine Auffangvorrichtung zur Entfernung niedergeschlagenen Desinfektionsmittels angeordnet ist.

Durch die Erfindung wird erreicht, daß nach einer Desinfektion sich Desinfektionsmittel und Feuchtigkeit in einem kälteren Bereich niederschlagen und von dort gezielt abgeführt werden können. Auf diese Weise ist es möglich, eine schnelle Trocknung und damit eine rasche Wiederinbetriebnahme herbeizuführen und kritische Bereiche der Vorrichtung wie das Mikrotom einer besonders effektiven Trocknung zu unterziehen, indem diese nicht gekühlt, sondern erwärmt werden. Dadurch wird bei einer erneuten Kühlung für den nächsten Arbeitsgang eine Eisbildung verhindert, welche das weitere Arbeiten beeinträchtigen oder unmöglich machen könnte. Insbesondere ist das Gerät nach einer Desinfektion relativ rasch und sicher wieder einsatzfähig.

Als Desinfektionsmittel können dabei die verschiedensten Mittel, meist als wäßrige Lösungen, eingesetzt werden. Beispielhaft seien Glutaraldehyd, Benzalkoniumchlorid, Formalin und Didecyldimethylammoniumcholorid genannt.

Die Temperaturdifferenz läßt sich sowohl durch die Kühlung eines Bereichs als auch durch die Erwärmung eines Bereichs erzielen. Zweckmäßig ist es dabei, vorhandene Kühl- oder Heizvorrichtungen einzusetzen. So kann vorgesehen sein, daß nach der Einwirkzeit die Temperatur des Kälteerzeugers des Kryostaten in einer Kühlphase unter 0°C abgesenkt wird, bis sich mindestens ein Großteil des Desinfektionsmittels am Kälteerzeuger niedergeschlagen hat, und daß der Kälteerzeuger danach aufgetaut wird, um das Desinfektionsmittel mittels einer Auffangvorrichtung aus der Kryostatkammer abzuführen. Auch ist normalerweise eine derartige Auffangvorrichtung unter dem Kälteerzeuger angeordnet, damit das Schmelzwasser von abgetautem Reif oder abgetautem Eis aus der Kryostatkammer entfernt werden kann.

Weiterhin ist es möglich, daß nach der Einwirkzeit das Mikrotom aufgeheizt wird. Durch eine solche Aufheizung wird das Desinfektionsmittel, beispielsweise die Desinfektionslösung, aus dem Mikrotom heraus und an kältere Teile der Vorrichtung überführt, wodurch erreicht wird, daß das Mikrotom für die nachfolgende Kühlphase im trockenen Zustand vorliegt. Die Temperaturdifferenz läßt sich natürlich sowohl durch eine Kühlung als auch durch eine Aufheizung erreichen, wobei jedoch die Kombination der beiden vorgenannten Maßnahmen am zweckmäßigsten ist. Die kälteren Bereiche sind dabei innerhalb der Kryostatkammer am zweckmäßigsten derart gewählt, daß niederschlagendes Desinfektionsmittel oder Feuchtigkeit gut aufgefangen und abgeleitet werden können.

Um auf jeden Fall eine völlige Trocknung des Mikrotoms zu erreichen, ist es zweckmäßig, daß die Aufheizung deutlich über die Umgebungstemperatur des Kryostaten geht.

Um auch eine möglichst wirksame Desinfektion zu erreichen, wird vorgeschlagen, daß das dampfförmige Desinfektionsmittel in die Kryostatkammer eingeblasen wird, wobei diese erzwungene Konvektion zu einer gleichmäßigen Benetzung aller Bauteile führt. Die Verdampfung des Desinfektionsmittels kann durch eine Heizung bewirkt werden, wobei jedoch vorzugsweise eine Verdampfung mittels Ultraschall erfolgt, da hierdurch ein gleichmäßiges Aerosol erzeugbar ist, ohne daß es zu einer Phasentrennung von Stoffen des Desinfektionsmittels kommt. Dies dient einer gleichmäßigen Desinfektionswirkung. Selbstverständlich kann das Desinfektionsmittel auch dampfförmig vorhanden sein.

Zweckmäßigerweise erfolgt nach der Entfrostungsphase eine Aufwärmung des Kryostaten mindestens auf die Umgebungstemperatur. An diese Aufwärmung schließt sich dann zweckmäßigerweise eine Temperaturausgleichszeit an, damit alle Bauteile auf gleicher Temperatur sind und dadurch während der Einwirkzeit des Desinfektionsmittels einer gleichmäßigen Desinfektion unterzogen werden. Zur Aufwärmung können verschiedene Heizungen eingesetzt werden, wobei zweckmäßigerweise eine im Mikrotom eingebaute Heizung herangezogen wird. Die Aufheizung über das Mikrotom verhindert dabei ein Betauen desselben und somit eine Lösungsmittelverdünnung während der eigentlichen nachfolgenden Desinfektion währen der Einwirkzeit, so daß eine einwandfreie Desinfektion des Mikrotoms erzielbar ist.

Vor der Bereitstellung des dampfförmigen Desinfektionsmittels sollte eine mechanische Entfernung von Schnittabfällen erfolgen. Dies kann manuell geschehen oder es kann vorgesehen sein, daß die Schnittabfälle abgesaugt werden. Dadurch wird vermieden, daß über die Schnittabfälle eine Kontamination in der Kryostatkammer verbleibt.

Weiterhin kann vorgesehen sein, daß dampfförmiges Desinfektionsmittel in eine Absauganlage eingesaugt wird, um auch diese ebenfalls zu desinfizieren. Durch das Einsaugen werden Schläuche, Filter, Pumpe und Absperrvorrichtungen der Absauganlage desinfiziert.

Weiterbildungen und Ausgestaltungen der Vorrichtung können den Weiterbildungen und Ausgestaltungen des Verfahrens entsprechen und umgekehrt, wobei die jeweils genannten Vorteile erzielbar sind.

Eine Weiterbildung der Vorrichtung sieht vor, daß die Steuerung derart ausgebildet ist, daß sie nach der Einwirkzeit die Temperatur des Kälteerzeugers des Kryostaten in einer Kühlphase unter 0°C absenkt, bis sich mindestens ein Großteil des Desinfektionsmittels am Kälteerzeuger niedergeschlagen hat und der Kälteerzeuger danach abgetaut wird, um das Desinfektionsmittel mittels der Auffangeinrichtung aus der Kryostatkammer abzuführen.

Weiterhin kann alternativ oder besser zusätzlich vorgesehen sein, daß das Mikrotom eine Heizung aufweist und die Steuerung derart eingerichtet ist, daß sie eine Aufheizung des Mikrotoms nach der Einwirkzeit veranlaßt. Dadurch wird, wie bereits beim Verfahren beschrieben, eine völlige Trocknung des Mikrotoms erzielt.

Zweckmäßigerweise weist der Kälteerzeuger ebenfalls eine Heizung auf, wobei die Steuerung derart ausgebildet ist, daß sie die Heizung zur Beschleunigung der Abtauung einschaltet. Dadurch läßt sich am Kälteerzeuger sublimierte Desinfektionslösung und Feuchtigkeit relativ schnell verflüssigen und abführen, wodurch das Gerät schnell wieder betriebsbereit wird.

Die Einrichtung zur Bereitstellung eines dampfförmigen Desinfektionsmittels weist zweckmäßigerweise eine Gebläse auf, das das dampfförmige Desinfektionsmittel in die Kryostatkammer einleitet. Damit läßt sich eine Zwangskonfevtion in der Kryostatkammer mit einer gleichmäßigen Verteilung des Desinfektionsmittels erzielen. Die Einrichtung zur Verdampfung des Desinfektionsmittels ist dabei zweckmäßigerweise mit einem Ultraschallaktuator ausgestattet, um die bereits oben erwähnte Verneblung des Desinfektionsmittels ohne Phasentrennung zu erzielen. Die Einrichtung zur zur Erzeugung des dampfförmigen Desinfektionsmittels wird zweckmäßigerweise mittels eines Vorratsbehälters mit Desinfektionsmittel versorgt. Dabei kann ein Ventil dafür sorgen, daß der Flüssigkeitsspiegel des Desinfektionsmittels in der Einrichtung zur Erzeugung des dampfförmigen Desinfektionsmittels regelbar ist.

Die Erfindung wird nachfolgend anhand beispielhafter Darstellungen der Zeichnung erläutert. Es zeigen
- **Fig.1**: eine beispielhafte Darstellung der Temperaturverläufe und Verfahrens- schritte eines erfindungsgemäßen Verfahrens und
- **Fig. 2**: ein Ausführungsbeispiel eines erfindungsgemäßen Mikrotom-Kryostaten.

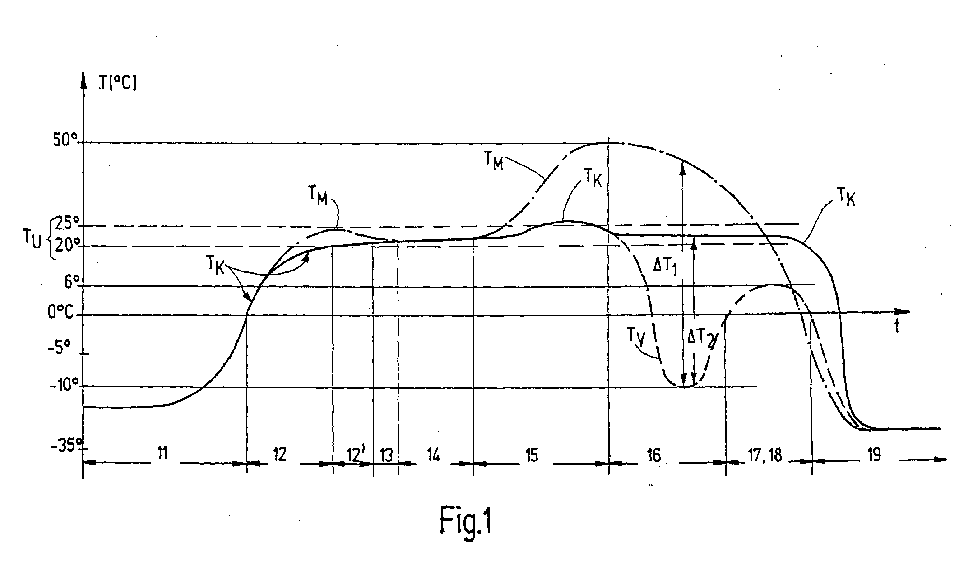

**Fig. 1** zeigt beispielhaft mögliche Temperaturverläufe des erfindungsgemäßen Verfahrens, wobei die Temperaturen T in °C gegen die Zeit t aufgetragen und mögliche Verfahrensschritte 11 bis 19 eingetragen sind.

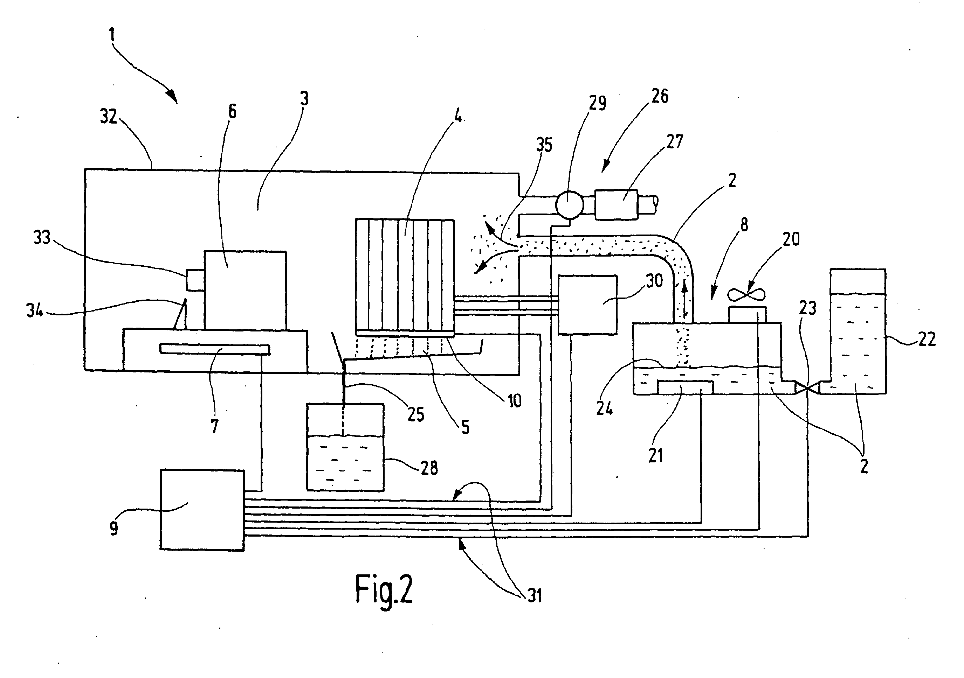

Ausgehend von einer Betriebstemperatur des Mikrotoms, die zwischen -5°C und -35°C liegt, wird eine Entfrostungsphase 11 eingeleitet und eine Aufwärmung 12 auf mindestens Umgebungstemperatur T_{U} vorgenommen, welche in etwa in einem Bereich von 20 bis 25°C liegt. Dieser Vorgang wird zweckmäßigerweise dadurch beschleunigt, daß die Heizung 7 des Mikrotom 6 betrieben wird, wobei die Aufheizung über das Mikrotom 6 den Vorteil hat, daß dieses nicht betaut wird. Dann kann es bei der nachfolgenden Desinfektion nicht vorkommen, daß das Desinfektionsmittel 2 durch diesen Tau verdünnt wird. Bezüglich der Vorrichtungsmerkmale wird auf die Figur 2 verwiesen.

Durch die Heizung des Mikrotoms 6 steigt die Temperatur T_{M} desselben schneller an als die Temperatur T_{K} der Kryostatkammer 3. Daher schließt sich zweckmäßigerweise an die Aufwärmung 12 eine Temperaturausgleichszeit 12' an. Wenn dann alle Bauteile ungefähr die gleiche Temperatur besitzen, erfolgt eine Bereitstellung 13 eines dampfförmigen Desinfektionsmittels 2, indem dieses in die Kryostatkammer 3 geblasen wird.

Nachdem alle Bauteile gleichmäßig mit Desinfektionsmittel 2 benetzt sind, wird eine Einwirkzeit 14 abgewartet, bis dann eine Aufheizung 15 des Mikrotoms 6 durch die Heizung 7 des Mikrotoms 6 eingeleitet wird. Dadurch steigt die Temperatur T_{M} des Mikrotoms 6 weiter an, beispielsweise auf 50°C. Dies dient dazu, daß Feuchtigkeit und Desinfektionslösung aus dem Mikrotom 6 heraus an die kälteren Teile der Kryostatkammer 3 überführt werden. Um eine gezielte Sublimation der Desinfektionslösung am Kälteerzeuger 4 des Kryostaten 1 herbeizuführen, wird dieser in einer Kühlphase 16 unter 0°C abgekühlt, vorzugsweise in dem Bereich von -10°C. Es entstehen dadurch Temperaturdifferenzen ΔT₁ und ΔT_{2,} wobei ΔT₁ die Differenz zwischen der Temperatur T_{M} des Mikrotoms 6 und der Temperatur T_{V} des Kälteerzeugers 4 und AT₂ die Differenz zwischen der Temperatur T_{K} der Kryostatkammer 3 und der Temperatur T_{V} des Kälteerzeugers 4 ist. Infolge dessen sublimiert das Desinfektionsmittel 2 und Feuchtigkeit an dem Kälteerzeuger 4 und schlägt sich dort als Reif und Eis nieder. Danach wird eine Abtauphase 17 des Kälteerzeugers 4 eingeleitet, in der die Abtauflüssigkeit abgeführt wird. Dieses Abführen 18 der Abtauflüssigkeit findet mittels einer Auffangvorrichtung 5 statt, welche die Abtauflüssigkeit aus der Kryostätkammer 3 herausführt. Dieses Abtauen 17 des Kälteerzeugers 4 wird zweckmäßigerweise durch eine Heizung 10 des Kälteerzeugers 4 unterstützt, wobei dieser beispielsweise auf +6°C geheizt wird. Ist die Abtauflüssigkeit abgeführt, sowie die Kryostatkammer 3 und vor allem das Mikrotom 6 trocken, so kann eine erneute Kühlung für eine Wiederinbetriebnahme 19 vorgenommen werden, damit der Mikrotom-Kryostat 1 wieder seine Betriebstemperatur für erneute Objektbearbeitung erreicht.

**Fig. 2** zeigt ein Ausführungsbeispiel eines erfindungsgemäßen Mikrotom-Kryostaten 1. Der Mikrotom-Kryostat 1 besteht aus einem gekühlten Gehäuse, das eine Kryostatkammer 3 umschließt und mittels einer Öffnung 32 öffenbar ist. In der Kryostatkammer 3 befindet sich ein Mikrotom 6 mit einem Objekthalter 33 und einem Messer 34 zur Herstellung der Schnitte. Die Kryostatkammer 3 wird mittels eines Kälteerzeugers 4, beispielsweise eines Verdampfers gekühlt, der an eine Kühlmittelversorgung 30 angeschlossen ist. Sowohl das Mikrotom 6 als auch der Kälteerzeuger 4 sind mit Heizungen 7 und 10 ausgestattet, um ein schnelles Abtauen und Aufheizen vornehmen zu können. Unterhalb des Kälteerzeugers 4 befindet sich eine Auffangeinrichtung 5, beispielsweise als Tropfschale ausgebildet, die über einen Ablauf 25 Auftauflüssigkeit aus der Kryostatkammer 3 heraus in einen Vorratsbehälter 28 abführt.

Weiterhin ist eine Einrichtung 8 zur Bereitstellung eines dampfförmigen Desinfektionsmittels 2 vorgesehen, die über ein Rohr in Richtung der Pfeile 35 dieses verdampfte Desinfektionsmittel 2 in die Kryostatkammer 3 blasen kann. Dazu ist ein Gebläse 20 vorgesehen sowie ein Ultraschallaktuator 21 zur Erzeugung des Aerosols aus dem flüssigen Desinfektionsmittel 2. Ein Vorratsbehälter 22 und ein Ventil 23 dienen dabei dazu, daß der Flüssigkeitsspiegel 24 des Desinfektionsmittels 2 in der Einrichtung 8 immer erhalten bleibt.

Erfindungsgemäß ist eine Steuerung 9 vorgesehen, welche mit der Heizung 7 des Mikrotoms 6, mit der Heizung 10 des Kälteerzeugers 4, mit dem Ultraschallaktuator 21, dem Gebläse 20 und der Kühlmittelversorgung 30 des Kälteerzeugers 4 verbunden ist. Dazu dienen Verbindungsleitungen 31. Die Steuerung 9 kann jedoch weiterhin mit nicht dargestellten Sensoren zur Erfassung von Temperaturen, Luftfeuchtigkeit, usw. verbunden sein, um die beschriebenen Temperaturen und Trocknungen effektiv regeln zu können. Weiterhin ist eine Verbindung zu einem Lüfter 29 einer Absauganlage 26 sowie mit dem Ventil 23 und weiteren Funktionselementen möglich.

Diese Verbindungsleitungen 31 dienen dazu, daß die Steuerung 9 die in Fig. 1 dargestellten Temperaturverläufe T_{K}, T_{M} und T_{V} sowie Verfahrensschritte 11 bis 19 steuern kann, indem sie die Heizungen 7 und/oder 10 sowie die Kühlung mittels des Kälteerzeugers 4 steuert. Außerdem steuert sie die Bereitstellung 13 des dampfförmigen Desinfektionsmittels 2 über die Einrichtung 8.

Weiterhin kann die Steuerung 9 dafür sorgen, daß dampfförmiges Desinfektionsmittel 2 in die Absauganlage 26 gesaugt wird, um dort beispielsweise den Lüfter 29, die Rohre und den Filter 27 zu desinfizieren.

Selbstverständlich ist sowohl die Darstellung der Temperaturverläufe T_{K}, T_{M} und T_{V} und der Arbeitsschritte 11 bis 19 der Fig. 1 sowie der Vorrichtungsmerkmale der Fig. 2 lediglich beispielhaft. Wesentlich sind die Temperaturdifferenzen ΔT₁ und ΔT₂ zum Niederschlag von Desinfektionsmittel 2 und Feuchtigkeit sowie deren Abfuhr. Es wäre auch eine alternative Ausgestaltung von Heizungen oder eine alternative Erzeugung von Temperaturdifferenzen innerhalb der Kryostatkammer 3 möglich, beispielsweise nur durch Heizung oder nur durch Kühlung oder es kann eine abweichende zeitliche Abfolge gewählt werden, bei der sich beispielsweise Verfahrensschritte 11 bis 19 teilweise überlagern können.

### Bezugszeichenliste

- 1: Mikrotom-Kryostat (Kryostat)
- 2: Desinfektionsmittel
- 3: Kryostatkammer
- 4: Kälteerzeuger (des Kryostaten), z. B. Verdampfer
- 5: Auffangeinrichtung
- 6: Mikrotom
- 7: Heizung des Mikrotoms
- 8: Einrichtung zur Bereitstellung eines dampfförmigen Desinfektionsmittels
- 9: Steuerung
- 10: Heizung des Kälteerzeugers
- 11: Entfrostungsphase
- 12: Aufwärmung auf mindestens Umgebungstemperatur
- 12': Temperaturausgleichszeit
- 13: Bereitstellung eines dampfförmigen Desinfektionsmittels
- 14: Einwirkzeit des Desinfektionsmittels
- 15: Aufheizung des Mikrotoms
- 16: Kühlung Kälteerzeuger
- 17: Abtauen Kälteerzeuger
- 18: Abführen der Abtauflüssigkeit
- 19: Kühlung für Wiederinbetriebnahme
- 20: Gebläse

- 21: Ultraschallaktuator
- 22: Vorratsbehälter für Desinfektionsmittel
- 23: Ventil
- 24: Flüssigkeitsspiegel
- 25: Ablauf
- 26: Absauganlage
- 27: Filter
- 28: Vorratsbehälter für Abtauflüssigkeit
- 29: Lüfter
- 30: Kühlmittelversorgung des Kälteerzeugers (Verdampfers)
- 31: Verbindungsleitungen zur Steuerung
- 32: Öffnung der Kryostatkammer
- 33: Objekthalter
- 34: Messer
- 35: Pfeile: Einblasen von dampfförmigen Desinfektionsmittel

- T: Temperatur in °C
- t: Zeit
- ΔT₁: Temperaturdifferenz zwischen Kälteerzeuger und Mikrotom
- ΔT₂: Temperaturdifferenz zwischen Kälteerzeuger und Kryostatkammer
- T_{U}: Umgebungstemperatur des Kryostaten
- T_{K}: Temperatur der Kryostatkammer
- T_{M}: Temperatur des Mikrotoms
- T_{V}: Temperatur des Kälteerzeugers (Verdampfers)

## Patentansprüche

1. Verfahren zur Desinfektion eines Mikrotom-Kryostaten (1) mit einer Entfrostungsphase (11), einer Bereitstellung (13) eines dampfförmigen Desinfektionsmittels (2) zur Einwirkung auf die geschlossene Kryostatkammer (3) und einer Einwirkzeit (14) für das Desinfektionsmittel (2),
**dadurch gekennzeichnet,**
**daß** nach der Einwirkungszeit (14) in der Kryostatkammer (3) eine Temperaturdifferenz (ΔT₁, ΔT₂) zwischen einem das Mikrotom (6) umfassenden Bereich und einem kälteren Bereich erzeugt und das im kälteren Bereich niedergeschlagene Desinfektionsmittel (2) abgeführt (18) wird.

2. Verfahren nach Anspruch 1,
**dadurch gekennzeichnet,**
**daß** nach der Einwirkzeit (14) die Temperatur (T_{V}) des Kälteerzeugers (4) des Kryostaten (1) in einer Kühlphase (16) unter 0°C abgesenkt wird, bis sich mindestens ein Großteil des Desinfektionsmittels (2) am Kälteerzeuger (4) niedergeschlagen hat, und daß der Kälteerzeuger (4) danach aufgetaut (17) wird, um das Desinfektionsmittel (2) mittels einer Auffangeinrichtung (5) aus der Kryostatkammer (3) abzuführen (18).

3. Verfahren nach Anspruch 1 oder 2,
**dadurch gekennzeichnet,**
**daß** nach der Einwirkzeit (14) das Mikrotom (6) aufgeheizt wird.

4. Verfahren nach Anspruch 3,
**dadurch gekennzeichnet,**
**daß** die Aufheizung (15) deutlich über die Umgebungstemperatur (T_{U}) des Kryostaten (1) geht.

5. Verfahren nach einem der Ansprüche 1 bis 4,
**dadurch gekennzeichnet,**
**daß** das dampfförmige Desinfektionsmittel (2) in die Kryostatkammer (3) eingeblasen wird.

6. Verfahren nach einem der Ansprüche 1 bis 5,
**dadurch gekennzeichnet,**
**daß** die Verdampfung des Desinfektionsmittels (2) mittels Ultraschall erfolgt.

7. Verfahren nach einem der Ansprüche 1 bis 6,
**dadurch gekennzeichnet,**
**daß** nach der Entfrostungsphase (11) eine Aufwärmung (12) des Kryostaten (1) mindestens auf Umgebungstemperatur (T_{U}) erfolgt.

8. Verfahren nach Anspruch 7,
**dadurch gekennzeichnet,**
**daß** sich an die Aufwärmung (12) eine Temperaturausgleichszeit (12') anschließt.

9. Verfahren nach Anspruch 7 oder 8,
**dadurch gekennzeichnet,**
**daß** die Aufwärmung (12) mittels der Heizung (7) des Mikrotoms (6) erfolgt.

10. Verfahren nach einem der Ansprüche 1 bis 9,
**dadurch gekennzeichnet,**
**daß** vor der Bereitstellung (13) des dampfförmigen Desinfektionsmittels (2) eine mechanische Entfernung von Schnittabfällen erfolgt.

11. Verfahren nach Anspruch 10,
**dadurch gekennzeichnet,**
**daß** die Schnittabfälle abgesaugt werden.

12. Verfahren nach einem der Ansprüche 1 bis 11,
**dadurch gekennzeichnet,**
**daß** dampfförmiges Desinfektionsmittel (2) in eine Absauganlage (26) eingesaugt wird, um auch diese zu desinfizieren.

13. Mikrotom-Kryostat (1) mit einer Kryostatkammer (3), in der sich ein Mikrotom (6) befindet, und mit einem Kälteerzeuger, umfassend eine Vorrichtung mit einer Einrichtung (8) zur Bereitstellung (13) eines dampfförmigen Desinfektionsmittels (2) und einer Steuerung (9), die derart ausgebildet ist, daß sie diese Bereitstellung (13) sowie eine Einwirkzeit (14) im Anschluß an eine Entfrostungsphase (11) veranlaßt,
**dadurch gekennzeichnet,**
**daß** die Steuerung (9) weiterhin derart ausgebildet ist, daß sie nach der Einwirkzeit (14) durch Heizung und/oder Kühlung in der Kryostatkammer (3) die Entstehung einer Temperatur differenz (ΔT₁, ΔT₂) zwischen einem das Mikrotom (6) umfassenden Bereich und einem kälteren Bereich veranlaßt und im kälteren Bereich eine Auffangvorrichtung (5) zur Entfernung niedergeschlagenen Desinfektionsmittels (2) angeordnet ist.

14. Mikrotom-kryostat nach Anspruch 13,
**dadurch gekennzeichnet,**
**daß** die Steuerung (9) derart ausgebildet ist, daß sie nach der Einwirkzeit (14) die Temperatur (T_{V}) des Kälteerzeugers (4) des Kryostaten (1) in einer Kühlphase (16) unter 0°C absenkt, bis sich mindestens ein Großteil des Desinfektionsmittels (2) am Kälteerzeuger (4) niedergeschlagen hat und der Kälteerzeuger (4) danach abgetaut (17) wird, um das Desinfektionsmittel (2) mittels der Auffangeinrichtung (5) aus der Kryostatkammer (3) abzuführen.

15. Mikrotom-kryostat nach Anspruch 13 oder 14,
**dadurch gekennzeichnet,**
**daß** das Mikrotom (6) eine Heizung (7) aufweist und die Steuerung (9) derart eingerichtet ist, daß sie eine Aufheizung (15) des Mikrotoms (6) nach der Einwirkzeit (14) veranlaßt.

16. Mikrotom-kryostat nach Anspruch 13, 14 oder 15,
**dadurch gekennzeichnet,**
**daß** der Kälteerzeuger (4) eine Heizung (10) aufweist, wobei die Steuerung (9) derart ausgebildet ist, daß sie die Heizung (10) zur Beschleunigung der Abtauung (17) einschaltet.

17. Mikrotom-kryostat nach einem der Ansprüche 13 bis 16,
**dadurch gekennzeichnet,**
**daß** die Einrichtung (8) zur Bereitstellung (13) eines dampfförmigen Desinfektionsmittels (2) ein Gebläse (20) aufweist, das letzteres in die Kryostatkammer (3) einleitet.

18. Mikrotom-kryostat nach einem der Ansprüche 13 bis 17,
**dadurch gekennzeichnet,**
**daß** die Einrichtung (8) zur Verdampfung des Desinfektionsmittels (2) mit einem Ultraschallaktuator (21) ausgestattet ist.

19. Mikrotom-kryostat nach einem der Ansprüche 13 bis 18,
**dadurch gekennzeichnet,**
**daß** die Einrichtung (8) mittels eines Vorratsbehälters (22) mit Desinfektionsmittel (2) versorgt wird.

20. Mikrotom-kryostat nach Anspruch 19,
**dadurch gekennzeichnet,**
**daß** der Flüssigkeitsspiegel (24) des Desinfektionsmittels (2) in der Einrichtung (8) mittels eines Ventils (23) regelbar ist.

21. Mikrotom-kryostat nach einem der Ansprüche 13 bis 20,
**dadurch gekennzeichnet,**
**daß** die Auffangvorrichtung (5) die vom Kälteerzeuger (4) abtropfende Flüssigkeit mittels eines Ablaufs (25) aus der Kryostatkammer (3) herausführt.

## Claims

1. A method for disinfecting a microtome/cryostat (1), comprising a defrosting phase (11), the provision (13) of a vaporous disinfectant (2) for reaction with the closed cryostat chamber (3) and a reaction period (14) for said disinfectant (2),
**characterized in that**
following said reaction period (14) in the cryostat chamber (3) a temperature difference (ΔT₁, Δ₂) between a region encompassing said microtome (6) and a colder region is produced and the disinfectant (2) condensed in the colder region is removed (18).

2. The method as defined in claim 1,
**characterized in that**
following said reaction period (14), the temperature (T_{V}) of the frigorific unit (4) in said cryostat (1) is lowered in a cooling phase (16) to below 0 °C, until at least a major portion of said disinfectant (2) has condensed on said frigorific unit (4), and the frigorific unit (4) is then defrosted (17) in order that said disinfectant (2) can be removed (18) from said cryostat chamber (3) by means of a receiving device (5).

3. The method as defined in claim 1 or claim 2,
**characterized in that**
following said reaction period (14) said microtome (6) is heated.

4. The method as defined in claim 3,
**characterized in that**
said heating (15) goes significantly beyond the ambient temperature (T_{U}) of said cryostat (1).

5. The method as defined in any one of claims 1 to 4,
**characterized in that**
said vaporous disinfectant (2) is blown into said cryostat chamber (3).

6. The method as defined in any one of claims 1 to 5,
**characterized in that**
volatilization of said disinfectant (2) is carried out by supersonic means.

7. The method as defined in any one of claims 1 to 6,
**characterized in that**
following the defrosting phase (11), said heating (12) of said cryostat (1) is effected at least to the ambient temperature (T_{U}).

8. The method as defined in claim 7,
**characterized in that**
said heating (12) is followed by a temperature compensation period (12').

9. The method as defined in claim 7 or claim 8,
**characterized in that**
said heating (12) is effected by means of the heater (7) pertaining to said microtome (6).

10. The method as defined in any one of claims 1 to 9,
**characterized in that**
prior to the provision (13) of said vaporous disinfectant (2), the waste cuttings are removed mechanically.

11. The method as defined in claim 10,
**characterized in that**
the waste cuttings are sucked off.

12. The method as defined in any one of claims 1 to 11,
**characterized in that**
vaporous disinfectant (2) is inspirated into an exhauster (26), in order to disinfect the same.

13. A microtome/cryostat (1) comprising a cryostat chamber (3), in which a microtome (6) is disposed, and comprising a frigorific unit, comprising a device having an assembly (8) for the provision (13) of a vaporous disinfectant (2) and having a control unit (9) adapted to cause said provision (13) of vaporous disinfectant and to provide a reaction period (14) following a defrosting phase (11),
**characterized in that**
said control unit (9) is further adapted such that following said reaction period (14) it creates, by heating and/or cooling within said cryostat chamber (3), a temperature difference (ΔT₁, AT₂) between a region encompassing said microtome (6) and a colder region, and in the colder region a receiving device (5) is disposed for the removal of condensed disinfectant (2).

14. The microtome/cryostat as defined in claim 13,
**characterized in that**
said control unit (9) is adapted such that, following said reaction period (14), it lowers the temperature (T_{V}) of the frigorific unit (4) in said cryostat (1) in a cooling phase (16) to below 0 °C, until at least a major proportion of said disinfectant (2) has condensed on said frigorific unit (4), after which the frigorific unit (4) is defrosted (17) for the purpose of removing said disinfectant (2) from said cryostat chamber (3) by means of said receiving device (5).

15. The microtome/cryostat as defined in claim 13 or claim 14,
**characterized in that**
said microtome (6) has a heating unit (7), and said control unit (9) is adapted such that it causes heating (15) of said microtome (6) following said reaction period (14).

16. The microtome/cryostat as defined in any one of claims 13, 14, and 15,
**characterized in that**
said frigorific unit (4) has a heating unit (10), said control unit (9) being adapted such that it switches on said heating unit (10) for the purpose of acceleration defrosting (17).

17. The microtome/cryostat as defined in any one of claims 13 to 16,
**characterized in that**
said assembly (8) comprises a ventilator (20) for the provision (13) of a vaporous disinfectant (2), which ventilator introduces the latter into said cryostat chamber (3).

18. The microtome/cryostat as defined in any one of claims 13 to 17,
**characterized in that**
said assembly (8) is provided with a supersonic actuator (21) for the purpose of volatilizing said disinfectant (2).

19. The microtome/cryostat as defined in any one of claims 13 to 18,
**characterized in that**
said assembly (8) is supplied with disinfectant (2) from a stock vessel (22).

20. The microtome/cryostat as defined in claim 19,
**characterized in that**
the liquid level (24) of said disinfectant (2) in said assembly (8) can be controlled by means of a valve (23).

21. The microtome/cryostat as defined in any one of claims 13 to 20,
**characterized in that**
said receiving device (5) guides the liquid dripping from said frigorific unit (4) away from said cryostat chamber (3) by means of a drain outlet (25).

## Revendications

1. Procédé pour la désinfection d'un cryostat de microtome (1) avec une phase de dégivrage (11), une préparation (13) d'un agent désinfectant (2) sous forme vapeur pour une action sur la chambre de cryostat fermée (3) et un temps d'action (14) pour l'agent désinfectant (2),
**caractérisé en ce qu'**après le temps d'action (14) dans la chambre de cryostat (3), il est généré une différence de température (ΔT₁, ΔT₂) entre une zone comprenant le microtome (6) et une zone plus froide et **en ce que** l'agent désinfectant (2) se précipitant dans la zone plus froide est évacué (18).

2. Procédé selon la revendication 1, **caractérisé en ce qu'**après le temps d'action (14), la température (T_{V}) du générateur de froid (4) du cryostat (1) est abaissée en dessous de 0°C dans une phase de refroidissement (16) jusqu'à ce qu'au moins une grande partie de l'agent désinfectant (2) se soit précipitée sur le générateur de froid (4) et que le générateur de froid (4) soit ensuite dégelé (17) pour évacuer (18) l'agent désinfectant (2) au moyen d'un dispositif de collecte (5) hors de la chambre de cryostat (3).

3. Procédé selon la revendication 1 ou 2, **caractérisé en ce qu'**après le temps d'action (14), le microtome (6) est chauffé.

4. Procédé selon la revendication 3, **caractérisé en ce que** le chauffage (15) monte nettement au-delà de la température ambiante (Tu) du cryostat (1).

5. Procédé selon l'une des revendications 1 à 4, **caractérisé en ce que** l'agent désinfectant sous forme vapeur (2) est soufflé dans la chambre de cryostat (3).

6. Procédé selon l'une des revendications 1 à 5, **caractérisé en ce que** l'évaporation de l'agent désinfectant (2) s'effectue par ultrason.

7. Procédé selon l'une des revendications 1 à 6, **caractérisé en ce qu'**après la phase de dégivrage (11), il se produit un réchauffement (12) du cryostat (1) au moins à a température ambiante (Tu).

8. Procédé selon la revendication 7, **caractérisé en ce que** le réchauffement (12) est suivi d'une période de compensation de température (12').

9. Procédé selon la revendication 7 ou 8, **caractérisé en ce que** le réchauffement (12) s'effectue au moyen du chauffage (7) du microtome (6).

10. Procédé selon l'une des revendications 1 à 9, **caractérisé en ce qu'**avant la préparation (13) de l'agent désinfectant (2) sous forme vapeur, il s'effectue une élimination mécanique de déchets de coupe.

11. Procédé selon la revendication 10, **caractérisé en ce que** les déchets de coupe sont aspirés.

12. Procédé selon l'une des revendications 1 à 6, **caractérisé en ce que** l'agent désinfectant (12) sous forme vapeur est aspiré dans une installation d'aspiration (26) pour la désinfecter également.

13. Cryostat de microtome (1) avec une chambre de cryostat (3), dans laquelle se trouve un microtome (6) et un générateur de froid, comprenant un dispositif avec un appareil (8) pour la préparation (13) d'un agent désinfectant (12) sous forme vapeur et une commande (9) qui est conçue de manière à provoquer cette préparation (13) ainsi qu'un temps d'action (14) à la suite d'une phase de dégivrage (11),
**caractérisé en ce que**
la commande (9) est conçue en outre de manière à déclencher après le temps d'action (14) par chauffage et/ou refroidissement dans la chambre de cryostat (3) la génération d'une différence de température (ΔT₁, ΔT₂) entre une zone comprenant le microtome (6) et une zone plus froide et **en ce qu'**un dispositif de collecte (5) pour l'élimination d'agent désinfectant précipité (2) est disposé dans la zone plus froide.

14. Cryostat de microtome selon la revendication 13, **caractérisé en ce que** la commande (9) est configurée de sorte qu'après le temps d'action (14), la température (Tᵥ) du générateur de froid (4) du cryostat (1) est abaissée en dessous de 0°C dans une phase de refroidissement (16) jusqu'à ce qu'au moins une grande partie de l'agent désinfectant (2) se soit précipitée sur le générateur de froid (4) et que le générateur de froid (4) soit ensuite dégelé (17) pour évacuer (18) l'agent désinfectant (2) au moyen d'un dispositif de collecte (5) hors de la chambre de cryostat (3).

15. Cryostat de microtome selon la revendication 13 ou 14, **caractérisé en ce que** le microtome (6) présente un chauffage (7) et la commande (9) est agencée de sorte qu'elle provoque un échauffement (15) du microtome (6) après le temps d'action (14).

16. Cryostat de microtome selon la revendication 13, 14 ou 15 **caractérisé en ce que** le générateur de froid (4) présente un chauffage (10), la commande (9) étant configurée de manière à mettre en route le chauffage (10) pour l'accélération du dégivrage (17).

17. Cryostat de microtome selon l'une des revendications 13 à 16, **caractérisé en ce que** l'appareil (8) de préparation (13) d'un agent désinfectant (2) sous forme vapeur présente un ventilateur (20) qui introduit ce dernier dans la chambre de cryostat (3).

18. Cryostat de microtome selon l'une des revendications 13 à 17, **caractérisé en ce que** l'appareil (8) pour l'évaporation de l'agent désinfectant (2) est muni d'un activateur d'ultrasons (21).

19. Cryostat de microtome selon l'une des revendications 13 à 18, **caractérisé en ce que** l'appareil (8) est alimenté en agent désinfectant (2) au moyen d'un réservoir (22).

20. Cryostat de microtome selon la revendication 20, **caractérisé en ce que** le niveau de liquide (24) de l'agent désinfectant (2) est réglable dans l'appareil (8) au moyen d'une vanne (23).

21. Cryostat de microtome selon l'une des revendications 13 à 20, **caractérisé en ce que** le dispositif de collecte (5) amène vers l'extérieur de la chambre de cryostat (3) à l'aide d'une vidange (25) le liquide goutant du générateur de froid (4).
